(19) Europäisches Patentamt
European Patent Office
Office européen des brevets

(11) Veröffentlichungsnummer: **0 308 614**
**A2**

(12) # EUROPÄISCHE PATENTANMELDUNG

(21) Anmeldenummer: **88111763.4**

(51) Int. Cl.⁴: **C07H 15/252 , A61K 31/70**

(22) Anmeldetag: **27.02.85**

(30) Priorität: **09.03.84 FR 8403634**

(43) Veröffentlichungstag der Anmeldung:
**29.03.89 Patentblatt 89/13**

(60) Veröffentlichungsnummer der früheren Anmeldung nach Art. 76 EPÜ: **0 156 172**

(84) Benannte Vertragsstaaten:
**AT BE CH DE GB IT LI LU NL SE**

(71) Anmelder: **LABORATOIRES HOECHST S.A.**
**TOUR ROUSSEL HOECHST 1 Terrasse Bellini**
**F-92800 Puteaux(FR)**

(72) Erfinder: **Florent, Jean-Claude**
**23 rue des Caussees**
**F-91940 Les Ulis(FR)**
Erfinder: **Monneret, Claude**
**9 avenue Lamoricière**
**F-75012 Paris(FR)**

(74) Vertreter: **Orès, Bernard et al**
**Cabinet ORES 6, Avenue de Messine**
**F-75008 Paris(FR)**

(54) **Neue Glykoside (Anthracycline) und Verwendung dieser Glykoside als Arzneimittel.**

(57) Vorliegende Erfindung betrifft neue Glykoside (Anthracycline), deren Herstellungsverfahren sowie die Verwendung dieser Glykoside als Arzneimittel.

EP 0 308 614 A2

## Neue Glykoside (Anthracycline) und Verwendung dieser Glykoside als Arzneimittel

Vorliegende Erfindung betrifft neue Glykoside (Anthracycline), deren Herstellungsverfahren sowie die Verwendung dieser Glykoside als Arzneimittel.
Gegenstand vorliegender Erfindung ist die Umwandlung der Isopropylidenanthracyclinone gemäss Hauptanmeldung n° 85 102 131.1 in Anthracycline durch Glykosidierung mittels 3,4-Di-O-acetyl-2,6-didesoxy-α-L-lyxohexosechlorid (24) nach der Koenigs-Knorr-reaktion, gemäss nachfolgendem Formelbild XII :

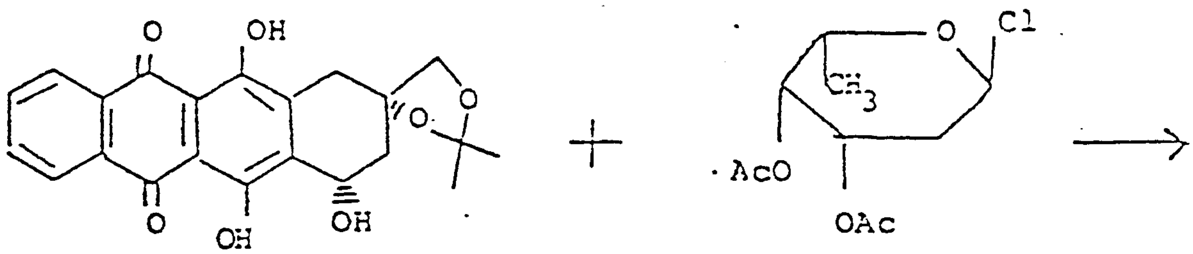

(23) (24)

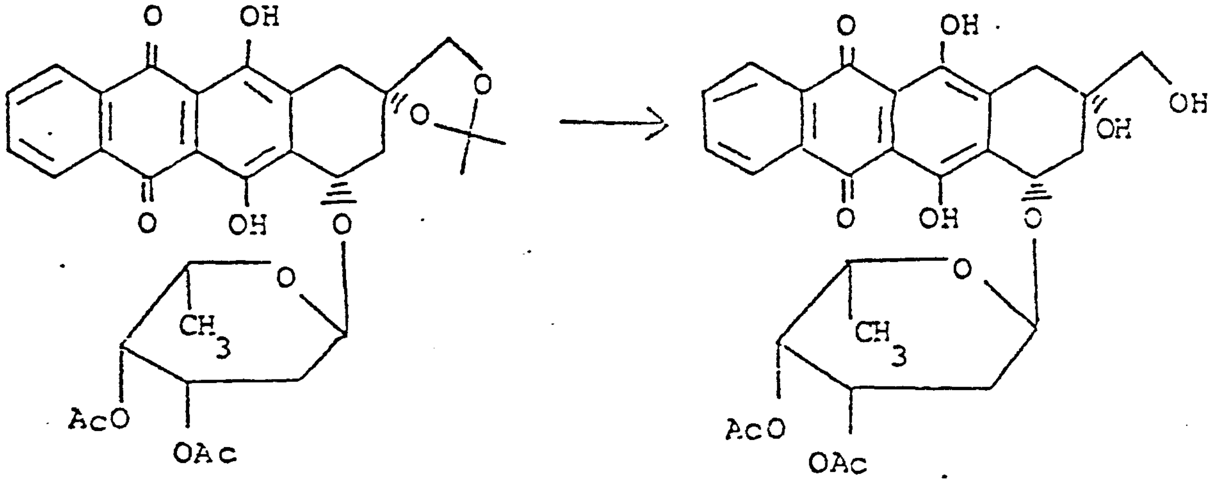

(25) (26)

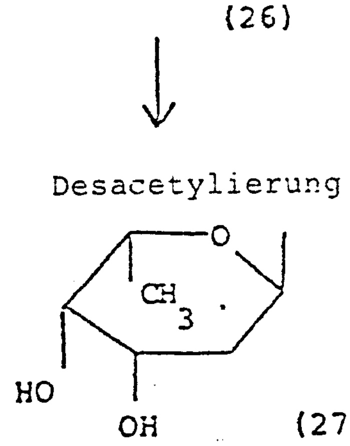


(27)

Formelbild XII

Neben den vorhergehenden Ausführungsformen umfasst die Erfindung ferner weitere, aus der nachfolgenden Beschreibung ersichtliche Ausführungsformen.

Gegenstand der Erfindung sind insbesondere die neuen Anthracycline und ihre Herstellungsverfahren sowie die Zusammensetzungen, insbesondere therapeutische Zusammensetzungen, in welche diese Derivate eingearbeitet werden.

Die Erfindung wird mit Hilfe der folgenden ergänzenden Beschreibung, die sich auf Ausfürungsbeispiele der erfindungsgemässen Verfahren bezieht, noch besser verdeutlicht.

BEISPIEL 1 :

1,2,3,4,6,11-Hexahydro-1(S),3(S),5,12-tetrahydroxy-3-C hydroxymethyl-3,13-0-isopropyliden-6,11-naphthacendion der Formel (23) :

1. Bei -10° C und dann 0° C :

das Aldehydderivat wird unter den zur Herstellung von 19 gemäss erste Teilanmeldung schon beschriebenen Bedingungen behandelt (300 mg Aldehyd, 40 ml THF/Methanol (1:1), 120 mg Natriumhydroxyd, 140 mg Natriumhydrosulfit une 10 ml Wasser). Dabei gewinnt man nach 2 Stunden bei 0° C 270 mg Rohprodukt. Dieses wird über Kieselsäure (Laufmittel : Toluol/Aceton 98:2) chromatographiert. Dabei erhält man nacheinander 65 g 14 und danach 60 mg 23 (Gesamtausbeute : 50 %).

2. Bei -40° C :

wird dieselbe Umsetzung bei -40° C durchgeführt, so findet man, dass laut DSC (Toluol/Aceton, 98 : 2) nur 23 vorliegt. So ergeben 300 mg 22 300 mg kristallinsiertes Rohprodukt, das aus Aceton/Methanolgemisch umkristallisiert wird. Dabei erhält man reines 23 als erstes Kristallisat (165 mg, 55 %) : Schmelzpunkt 270-274°, $(\alpha)_D^{20}$ + 96° (c l, Chloroform) ;

IR $\nu$ max $CHCl_3$ : 3500 (OH), 1625 und 1590 $cm^{-1}$ (Chinon mit Wasserstoffbrücken)

NMR ($CDCl_3$, 270 MHz) :

13,32 (1H, s) und 13,12 (1H, s) (2 phenolische OH mit Wasserstoffbrücken). 8,17 (2H, m) und 7,72 (2H, m) (System AA′ und BB′) (4H aromatisch). 5,14 (1H, m, J = 10, J′ = 5, J″ = 2, H-1). 4,08 (1H, d, J = 10, OH verschwindet nach Deuterierung). 3,96 (2H, s, $CH_2$-13). 3,21 (1H, dd, J = 18,5 J′ = 1,5, H-4e) und 2,70 (1H, d, J = 18,5, H-4a). 2,43 (1H, m, J = 14, J′ = 2, J″ = 1,5, H-8e) und 1,98 (1H, dd, J = 14, J′ = 5, H-8a). 1,48 (3H, s) und 1,43 (3H, s) (2 $CMe_2$).

Massenspektrum (DCI/$NH_3$) : m/z (M + 1, 45 %), 396 ($M^+$, 100 %), 381 (M - 15, 20 %), 320 (20 %).

Analyse :

Berechnet für $C_{22}H_{20}O_7$ (396,38) % :

C 66,66 H 5,09, O 28,25

Gefunden :

C 66,72, H 5,18 O 27,98.

BEISPIEL 2 :

1-O-(3′,4′-Di-O-acetyl-2′,6′-didesoxy-α-L-lyxo-hexapyranosyl)-1,2,3,4,6,11-hexahydro-1(S),3(S),5,12-tetrahydroxy-3-C-hydroxymethyl-3,13-O-isopropyliden-6,11-naphtacendion der Formel 25 :

Zu einer Lösung von 23 (75 mg, 0,19 mMol) in trockenem Dichlormethan (25 ml) gibt man gelbes Quecksilberoxyd (600 mg), Mercuribromid (210 mg) und Molekularsieb 4A (900 mg). Nach 1 Stunde Rühren bei Raumtemperatur versetzt man mit dem Chlorzucker 24 (125 mg, 0,50 mMol) in Lösung in 10 ml $CH_2Cl_2$. Nach 12 Stunden Rühren filtriert man das Reaktionsgemisch und extrahiert das Filtrat mit Dichlormethan mit nachfolgendem Waschen mit gesättigter Natriumbicabonatlösung und dann mit Wasser. Nach Trocknen und Abdampfen des Lösungsmittels bei vermindertem Druck erhält man einen Rückstand von ungefähr 200 mg Gewicht. Chromatographie Über Kieselsäure (Laufmittel : Toluol/Aceton, 95:5) liefert 100 mg reines kristallisierte 25 (87 % Ausbeute). Zur Analyse wird eine Probe aus Hexan/Aceton umkristallisert : Schmelzpunkt 199-200° ; $(\alpha)_D^{20}$ + 105° (c 0,04, Chloroform) ;

IR $\nu$ max $CHCl_3$ : 1040, 1225 und 1775 (Ester-CO), 1625 und 1590 (Chinon mit Wasserstoffbrücken).

NMR ($CDCl_3$, 270 MHz) :

13,58 (1H, s) und 13,30 (1H, s) (2 phenolische OH mit Wasserstoffbrücken). 8,28 (2H, m) und 7,80 (2H, m) (System AA′ und BB′, 4 H aromatisch). 5,58 (1H, d, J = 3, J′ < 1, H-4′). 5,24 (1H, m, H-3′). 5,20 (1H, s verbreitert, Halbwerts-J = 5, H-1). 5,03 (1H, t, J = 5, J′ = 4, H-1′). 4,40 (1H, q, J = 6,5, J′ < 1, H-5′). 3,95 (1H, d) und 3,85 (1H, d) (System AB, J = 9, $CH_2$-13). 3,27 (1H, d) und 2,80 (1H, d) (System AB, J = 19, $CH_2$-4). 1,90-2,35 (4H, m, $CH_2$-2 und 2′). 2,16 (3H, s) und 1,89 (3H, s) (2 OAc). 1,48 (6H, s, $CMe_2$); 1,21 (3H, d, J = 6,5 $CH_3$-6′).

Massenspektrum DCI/$NH_3$ : m/z 628 (M + 18, 10 %), 440 (10), 396 (30), 379 (15), 250 (30), 183 (60), 155 (100 %).

Analyse :

Berechnet für $C_{32}H_{34}O_{12}$ (610,59) % :

C 62,94, H 5,61, O 31,44

Gefunden :

C 62,78 H 5,68, O 31,58.

BEISPIEL 3 :

1-O-(3′,4′-Di-O-acetyl-2′,6′-didesoxy-α-L-lyxo-hexapyranosyl)-1,2,3,4,6,11-hexahydro-1(S),3(S),5,12-tetrahydroxy-3-C-hydroxymethyl-6,11-naphthacendion der Formel 26 :

Man rührt das Glykosid 25 (40 mg) 4 Stunden lang bei Raumtemperatur in 0,2n methanolischer Salzsäure (30 ml). Dann giesst man das Reaktionsgemisch in gesättigte Natriumbicarbonatlösung (15-20 ml) und extrahiert in üblicher Weise mit Dichlormethan. Nach Abdampfen des Lösungsmittels bei vermindertem Druck gewinnt man 40 mg Rohprodukt. Dieses wird über Kieselsäure chromatographiert (Laufmittel : $CH_2Cl_2$/Methanol 98:2). Nacheinander gewinnt man dabei 22 mg Ausgangsprodukt 25 und 18 mg 26. Behandelt man die 22 mg 25 in der gleichen Weise, so erhält man ein zweites Kristallisat 26 (8 mg), d.h. insgesamt 26 mg (≈ 65 %). Eine Probe wird zur Analyse aus Hexan/Aceton 1:1 umkristallisiert :

Schmelzpunkt 207-209° ; $(\alpha)_D^{20}$ + 91° (c 0,05, Chloroform) ;

IR ν max $CHCl_3$ : 3500 (OH), 1740, 1200-1250, 1020 (Ester), 1625 und 1590 $cm^{-1}$ (Chinon mit Wasserstoffbrücken)

NMR ($CDCl_3$, 270 MHz) :

13,60 (1H, s) und 13,30 (1H, s) (2 phenolische OH mit Wasserstoffbrücken). 8,28 (2H, m) und 7,80 (2H, m) (System AA′ und BB′, 4H aromatisch). 5,68 (1H, d, J = 3, H-4′). 5,32 (1H, t verbreitert, J = 4, J′ = 1, H-1′). 5,26 (1H, s verbreitert, Halbwerts-J 5, H-1). 5,08 (1H, m, H-3′). 4,34 (1H, q, J = 6, J′<1, H-5′). 3,76 (1H, d) und 3,54 (1H, dd) (System ABX, J = 10, J′ = 8, $CH_2$-13). 3,28 (1H, d) und 2,62 (1H, d) (System AB, J = 19, $CH_2$-4). 2,35-1,90 (4H, m, $CH_2$-2′ und 2). 2,20 (3H, s) und 1,98 (3H, s) (2 OAc). 1,23 (3H, d, J = 6,5, $CH_3$-6′).

Massenspektrum : (DCI/$NH_3$) : m/z 588 (M + 18 10 %), 356 (15), 155 (100).

Wie aus dem obigen ersichtlich ist, beschränkt sich die Erfindung Keineswegs auf solche Ausführungs- und Anwendungsformen, wie oben näher beschrieben, sondern umfasst sämtliche Varianten, die einem Fachman naheliegen könnten, ohne vom Umfang oder dem Gedanken der Erfindung abzuweichen.

## Ansprüche

1. Verfahren zur Umwandlung der Isopropylidenanthracyclinone in Anthracycline durch Glykosidierung mittels 3,4-Di-O-acetyl-2,6-didesoxy-α-L-lyxohexosechlorid (24) nach der Koenigs-Knorr-reaktion, gemäss nachfolgendem Formelbild XII ;

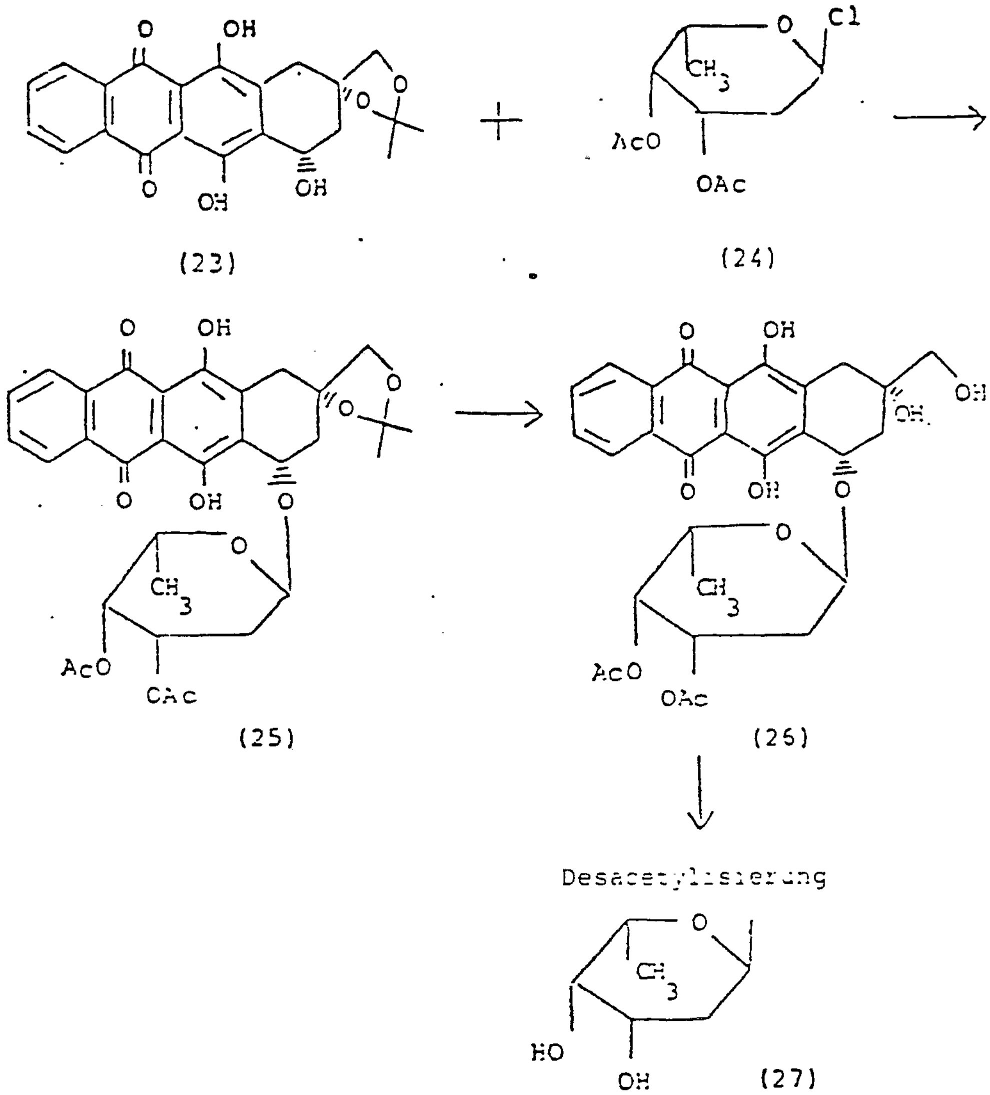


(23) (24)

(25) (26)

(27)

2. Anthracyclin, bestehend aus 1-O-(3′,4′-Di-O-acetyl-2′,6′-didesoxy-α-L-lyxo-hexopyranosyl)-1,2,3,4,6,11-hexahydro-1(S),3(S),5,12-tetrahydroxy-3-C-hydroxymethyl-3,13-O- isopropyliden-6,11-naphthacendion der nachfolgenden Formel (25) :

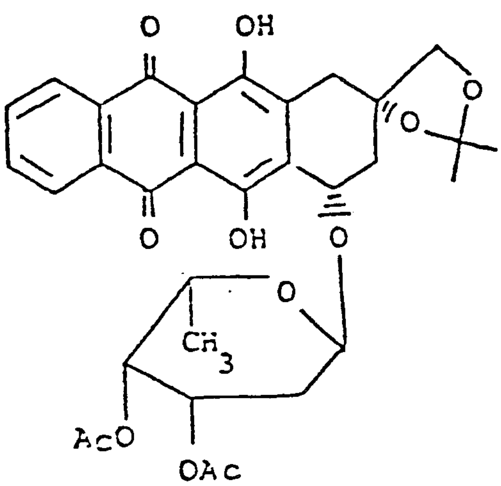

(25)

3. Anthracyclin, bestehend aus 1-O-(3′,4′-Di-O-acetyl-2′,6′-didesoxy-α-L-lyxo-hexopyranosyl)-1,2,3,4,6,11-hexahydro-1(S),3(S),5,12-tetrahydroxy-3-C-hydroxymethyl- 6,11-naphthacendion der nachfolgenden Formel (26) :

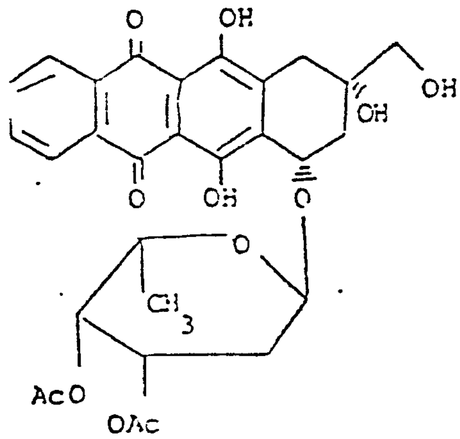

(26)

4. Anthracyclin, bestehend aus 1-O-(2′,6′-Didesoxy-α-L-lyxo-hexopyranosyl)-1,2,3,4,6,11-hexahydro-1-(S),3(S),5,12-tetrahydroxy-3-C-hydroxymethyl-6,11-naphthacendion der nachfolgenden Formel (27) :

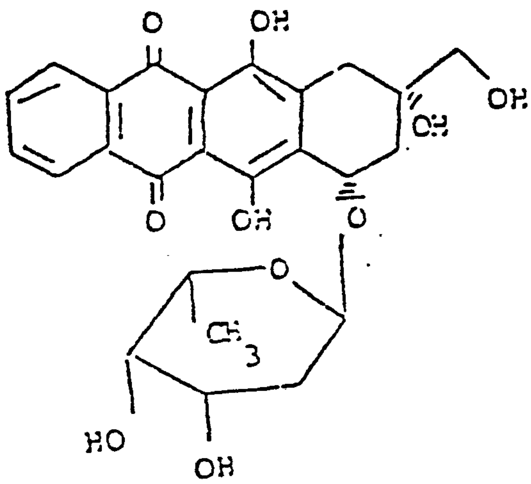

(27)